(19) **Europäisches Patentamt European Patent Office Office européen des brevets**

(11) **EP 3 372 984 B1**

(12) **EUROPEAN PATENT SPECIFICATION**

(45) Date of publication and mention
of the grant of the patent:
**12.08.2020 Bulletin 2020/33**

(21) Application number: **16881643.7**

(22) Date of filing: **16.12.2016**

(51) Int Cl.:
*G01N 15/00* (2006.01)      *G01N 15/06* (2006.01)
*C12M 3/00* (2006.01)       *G01N 1/02* (2006.01)
*G01N 1/28* (2006.01)       *C12M 1/00* (2006.01)
*G01N 1/22* (2006.01)       *G01N 1/44* (2006.01)
*G01N 1/24* (2006.01)

(86) International application number:
**PCT/JP2016/087518**

(87) International publication number:
**WO 2017/115667 (06.07.2017 Gazette 2017/27)**

(54) **GAS-BORNE FINE PARTICLE MEASURING INSTRUMENT AND CLEAN ENVIRONMENTAL DEVICE**

MESSINSTRUMENT FÜR GASGETRAGENEN FEINSTAUB UND VORRICHTUNG FÜR SAUBERE UMWELT

INSTRUMENT DE MESURE DE PARTICULES FINES EN SUSPENSION DANS UN GAZ ET DISPOSITIF ENVIRONNEMENTAL PROPRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2015 JP 2015255817**

(43) Date of publication of application:
**12.09.2018 Bulletin 2018/37**

(73) Proprietor: **PHC Holdings Corporation Tokyo 105-8433 (JP)**

(72) Inventors:
• **SEKINE, Hironobu
Toon-shi, Ehime, 791-0395 (JP)**
• **HIRAI, Hiroki
Toon-shi, Ehime, 791-0395 (JP)**

• **KOBAYASHI, Koichi
Toon-shi, Ehime, 791-0395 (JP)**
• **NASU, Hiroshi
Toon-shi, Ehime, 791-0395 (JP)**

(74) Representative: **Grünecker Patent- und Rechtsanwälte
PartG mbB
Leopoldstraße 4
80802 München (DE)**

(56) References cited:
**EP-A1- 0 967 268      EP-A2- 2 692 851
JP-A- H0 445 784      JP-A- S60 141 279
JP-A- 2002 340 778    JP-A- 2002 360 428
JP-A- 2006 166 748    JP-A- 2009 002 711
JP-A- 2010 154 792    JP-A- 2017 055 742
JP-U- S57 182 149     US-A1- 2010 313 963**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# Description

[Technical Field]

The present disclosure relates to an air particle measurement apparatus and clean environment equipment.

[Background Art]

For example, in cell culture work for regenerative medicine or other work, a particle measurement apparatus or a particle counter is used for measuring particles in working environment, as an indicator of cleanliness (for example, JP 2006-058239 A) .

EP 2692851 A2 relates to a sensor unit and a constant-temperature device using said sensor unit. The sensor unit comprises a block having an interior space communicated with an incubation chamber, an air-flow generation means for sucking atmosphere from the incubation chamber to the interior space and for discharging the atmosphere into the incubation chamber, an atmosphere measuring means for measuring the atmosphere sucked into the interior space, filters arranged between the incubation chamber and the interior space and a heater for heating the atmosphere sucked through the filter.

EP 0976268 A1 relates to an apparatus wherein due condensation of the gas to be measured is avoided by heating the gas to the respective temperature. The apparatus comprises a means provided for reducing the moisture content, for example by de-humidifying or heating the atmosphere, in order to avoid an implementation of means for reducing the moisture content.

JP 2010-154792 A relates to an apparatus comprising a heating means, which allows for the temperature in the bypass route to the detection unit to be even higher than the temperature in the respective chamber.

JP 2009-002711 A relates to sample concentration method characterized by removing water of a smaple by a concentration trap mechanism and sending it to an analyzer.

US 2010/313963 A1 discloses a gas mixer and sampler system for a controlled atmosphere laboratory chamber wherein the mixer and sampler system has a sampler remote from the chamber and a gas supply arrangement, including a mixer remote from the chamber. The sampler consists of a gas sensor arrangement for sensing a first and a second gas. A processor output from the gas sensors to determine the relative concentrations of the first and second gases, and determine proportions and flow rate for gases to be supplied to the chamber to meet a determined profile. The mixer and sampler are provided externally of the chamber at a location spaced from the chamber.

[Summary of Invention]

[Technical Problem]

Such a particle measurement apparatus is usually used for measuring particles in the air in an ordinary state (temperature, humidity). However, when measuring particles in the gas in the clean environment equipment, such as an isolator or an incubator, which temporarily or constantly has a high humidity, a common particle measurement apparatus may not be able to accurately measure the particles, due to condensation of the moisture particles in the air or condensation in a sampling tube.

[Solution to Problem]

An embodiment of the present disclosure to solve such a problem is an air particle measurement apparatus connected to a chamber, the chamber temporarily or constantly maintained at a humidity higher than an outside air humidity, the air particle measurement apparatus comprising: a measurement unit configured to measure particles in the drawn gas; a suction pipe through which the gas drawn from an interior of the chamber is transported to the measurement unit, the suction pipe connecting a mounting unit of the chamber and the measurement unit; a pump configured to suction the gas so as to be transported from the chamber to the measurement unit through the suction pipe; and a heating unit configured to heat the gas in a path upstream from the measurement unit, wherein the suction pipe is formed of a hygroscopic resin or wherein the apparatus comprises a removal unit configured to remove moisture in the gas by condensing moisture contained in the gas in the suction pipe, and wherein the heating unit has a structure to cover the suction pipe upstream from the measurement unit.

The present invention also provides a clean environment equipment configured to be detachably attached with an air particle measurement apparatus, the air particle measurement apparatus configured to measure particles in gas, the clean environment equipment comprising: a chamber temporarily or constantly maintained at a humidity higher than an outside air humidity; a connecting portion to detachably connect the chamber and the air particle measurement apparatus; and a heating unit configured to heat the gas flowing through the connecting portion, wherein the air particle measurement apparatus is the afore-mentioned air particle measurement apparatus.

[Advantageous Effects of Invention]

According to the present disclosure, it is possible to accurately measure particles in gas even when measuring the particle in the gas temporarily or constantly having a high humidity.

[Brief Description of the Drawings]

FIG.1 is a diagram schematically illustrating clean environment equipment according to a first embodiment.
FIG. 2 is a diagram schematically illustrating clean environment equipment according to a second embodiment.
FIG. 3 is a diagram schematically illustrating clean environment equipment according to an example not being part of the present invention.

[Description of Embodiments]

At least the following matter will become clear from the descriptions of the present specification with reference to the accompanying drawings.

[First Embodiment]

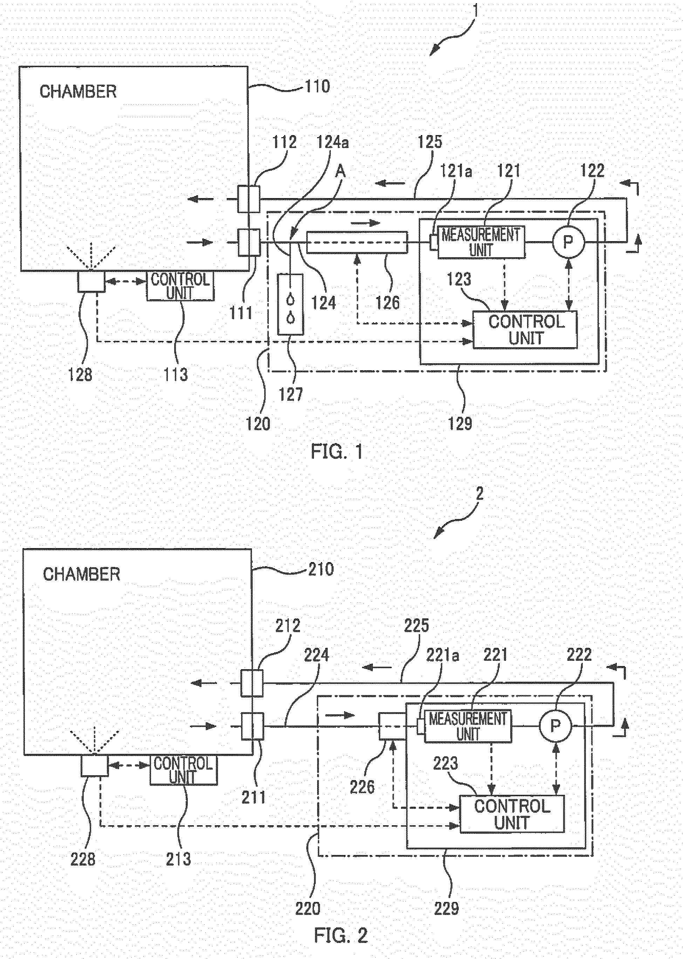

Clean environment equipment 1 according to a first embodiment will be described with reference to FIG. 1. FIG. 1 is a diagram schematically illustrating the clean environment equipment 1 according to the first embodiment.

Configuration of clean environment equipment

As illustrated in FIG. 1, the clean environment equipment 1 includes: a chamber 110; a supply unit 128 configured to supply water vapor or mist-like moisture to increase the humidity in the chamber 110; and an air particle measurement apparatus 120 connected to the chamber 110.

The chamber 110 is, for example, an isolator or an incubator, and provides a closed work space in which work on a biological sample (e.g., cell culture) is performed. In an embodiment according to the present disclosure, water vapor or mist-like moisture is supplied to the chamber 110 by the supply unit 128. The supply unit 128 is controlled by a control unit 113 of the clean environment equipment 1. The water vapor or mist-like moisture includes particles obtained, using a sprayer, by spraying humidifying water, sterilizing gas, and sterile liquid, which are used to humidify or sterilize the interior of the chamber 110. Here, the moisture includes not only water but also aqueous solution.

The interior of the chamber 110 is temporarily or constantly maintained at a humidity higher than the outside air humidity by the supply unit 128. For example, in the case of an isolator, the supply unit 128 is configured to supply sterilizing gas obtained by vaporizing sterile liquid. Then, in a sterilizing process, it is necessary to condense the sterilizing gas on the inner wall, and thus the internal humidity temporarily reaches about 100%. In the case of an incubator, the supply unit 128 is a water storage portion disposed in the interior to store humidifying water. The water storage portion is heated by a heater such that the internal humidity is constantly maintained at about 95%. In such clean environment equipment, since the interior has a humidity equal to or greater than 90%, the technique according to the present disclose is particularly effective, however, it is effective at least in equipment in which the humidity is temporarily or constantly maintained greater than the outside air humidity. In such a chamber 110, since it is necessary to maintain clean work environment, the particles in the gas is measured by the air particle measurement apparatus 120 so that the particles contained in the gas within the work space do not exceed the predetermined value. Note that the chamber 110 includes a mounting unit 111 to connect a suction pipe 124, and a mounting unit 112 to connect a discharge pipe 125.

The air particle measurement apparatus 120 includes, as illustrated in FIG. 1, a measurement unit 121, a pump 122, a control unit 123, the suction pipe 124, the discharge pipe 125, a heater 126, and a removal unit 127. The measurement unit 121, the pump 122, and the control unit 123 are stored in a housing 129. Note that the suction pipe 124, the discharge pipe 125, the heater 126, and the removal unit 127 may be components attached to the chamber 110.

The measurement unit 121 is configured to measure the particles in the drawn gas. The measurement unit 121 is, for example, a light scattering sensor, and is configured to detect particles by irradiating the particles drawn in the measurement unit 121 with laser light and capturing the scattering of the light using the sensor.

The gas inlet port of the measurement unit 121 is provided with a flowmeter 121a. The flowmeter 121a is configured to measure the flow rate of the gas drawn into the measurement unit 121, and output a measurement signal to the control unit 123. Such a measurement signal is used for the control unit 123 to control the suction amount of the pump 122. Note that the flowmeter 121a may be provided downstream from the measurement unit 121.

The suction pipe 124 connects the mounting unit 111 of the chamber 110 and the measurement unit 121, and the gas drawn from the interior of the chamber 110 is transported to the measurement unit 121.

The pump 122 is, for example, a diaphragm pump or a rotary pump, and is configured to draw the gas to be transported from the chamber 110 through the suction pipe 124 to the measurement unit 121. In an embodiment of the present disclosure, the pump 122 is connected to the measurement unit 121 on the downstream side, but may be connected to the measurement unit 121 on the upstream side. Note that the operation of the pump 122 will be described later in connection with the control unit 123.

The discharge pipe 125 connects the pump 122 and the mounting unit 112 of the chamber 110, and transports the gas discharged from the pump 122 to the chamber 110. Thus, as illustrated by arrows in FIG. 1, the gas within the chamber 110 is drawn out from the mounting unit 111, and is returned into the chamber 110 from the mounting unit 112, through the suction pipe 124, the measurement unit 121, the pump 122, and the discharge pipe 125. That is, the suction pipe 124, the measurement unit 121, the pump 122, and the discharge pipe 125 form a path through which the gas flows.

The heater 126 is an example of a heating unit, and is configured to heat the gas in the path upstream from the measurement unit 121. In the first embodiment, the heater 126 has a structure to substantially completely cover the suction pipe 124. However, the heater 126 may have a structure to partially cover the suction pipe 124.

The temperature of the heater 126 is controlled by the control unit 123. For example, a temperature sensor (not shown) to measure the temperature of the gas is provided in the path downstream from the heater 126. The control unit 123 controls the temperature of the heater 126 based on the temperature of the gas measured by the temperature sensor and the preset temperature. The preset temperature is, for example, 100°C or may be set by a user.

The removal unit 127 is provided upstream from a heating unit 126, and is configured to remove the moisture in the gas by condensing the moisture in the gas in the suction pipe 124. In the first embodiment, the removal unit 127 is provided in the path between the mounting unit 111 and the heater 126.

The removal unit 127 may be constituted by, for example, a branch pipe 124a branched vertically downward from a position A in the suction pipe 124, and a container to receive water droplets dripping from the lower end of the branch pipe 124a. For example, assuming that the temperature within the chamber 110 is 37°C, and the temperature of the outside air is 25°C, the gas within the chamber 110 having a high humidity is cooled to condense in the suction pipe 124. Such moisture generated as a result of condensation drops from the branch pipe 124a to the container to be stored, while the gas with reduced moisture flows toward the heater 126.

Alternatively, the removal unit 127 may be a condenser to condense moisture in the gas by heat exchange of the gas in the path with cooling water.

The control unit 123 is configured to control driving of the pump 122 so as to adjust the flow rate of the gas drawn into the measurement unit 121. For example, the control unit 123 controls the pump 126 so that the flow rate of the gas drawn in the measurement unit 121 when the heater 126 is operating to generate a first amount of heat is greater than the flow rate when the heater 126 is operating to generate a second amount of heat smaller than the first amount of heat or the flow rate when the heater 126 is stopped. Note that the control unit 123 may be independent of the control unit 113 of the clean environment equipment 1, or may be in cooperation therewith. Further, the control unit 123 and the control unit 113 may be integrated.

The amount of suction of the pump 122 is adjusted based on a gas state equation. For example, the following Expression 1 holds from the gas state equation.

$$P_1 \times V_1 / T_1 = P_2 \times V_2 / T_2 \dots \quad \text{Expression 1}$$

where when the pressure, volume, and temperature of the gas before being heated by the heater 126 are $P_1$, $V_1$, and $T_1$, respectively, and the pressure, volume, and temperature of the gas after being heated are $P_2$, $V_2$, and $T_2$, respectively.

In accordance with this Expression 1, when assuming that the pressure is constant ($P_1 = P_2$), 28.32 liters of air at 25°C expands to 35.45 liters when heated to 100°C. Thus, for example, in the case where the measurement value corresponding to the number of the particles in the predetermined volume of the gas at 25°C is required regardless of whether being heated by the heater 126, and if the gas at 25°C flowing through the suction pipe 124 is heated to 100°C by the heater 126, the suction amount of the pump 122 is adjusted to be 1.25 times of the suction mount of the pump 122 when the gas is not heated by the heater 126. Alternatively, assuming that the suction amount of the pump 122 is constant, the time interval at which the measurement unit 121 measures is adjusted to be 1.25 times of the time interval at which the measurement unit 121 measures when the gas is not heated by the heater 126.

Further, the control unit 123 can monitor the operational status of the supply unit 128, and the control unit 123 can also control the suction amount of the pump 122 according to the operation of the supply unit 128. In specific, the pump 122 may be driven such that the flow rate of the gas drawn into the measurement unit 121 when water vapor or mist-like moisture is being supplied from the supply unit 128 becomes smaller than the flaw rate of the gas when water vapor or mist-like moisture is not supplied from the supply unit 128. For example, the control unit 123 may stop the pump 122 when humidifying water and/or sterile liquid is sprayed or sterilizing gas is supplied from the supply unit 128. That is, when humidification and sterilization are performed in the chamber 110, suction of the gas by the pump 122 may be stopped. Note that the control unit 123 may control the amount of suction of the pump 122 according to signals from the control unit 113 that controls the operation of the supply unit 128.

Further, the control unit 123 has a measurement mode and a reset mode (aeration mode). The measurement mode corresponding to a first mode is a mode of driving the pump 122 so that the flow rate of the gas drawn into the measurement unit 121 becomes a predetermined flow rate (first flow rate) corresponding to the temperature of the heater 126, so as to perform measurement with the measurement unit 121. The reset mode corresponding to a second mode is a mode of driving the pump 122 so that the gas at a flow rate (second flow rate) greater than the gas flow rate in the measurement mode flows into the measurement unit 121, so as to return the measurement unit 121 to its initial state. The reset mode is executed, for example, once a day. Alternatively, the reset mode may be executed to quickly remove the humidifying water, sterile liquid, sterilizing gas from the path of the gas after humidification or sterilization in the chamber 110.

Operation of clean environment equipment

The operation of the clean environment equipment 1 including the above-described configuration will be described. The operation of the clean environment equipment 1 is broadly classified into the operation when measuring the particles in the gas, the operation when the measurement unit 121 is reset to its initial state, and the operation when the interior of the chamber 110 is sterilized, and will be described below in sequence.

When measuring particles

As described above, the gas in the chamber 110 is drawn by the pump 122 through the suction pipe 124 into the measurement unit 121, and the particles therein are measured at the measurement unit 121. At this time, the gas having a high humidity drawn from the chamber 110 is heated to the desired temperature by the heater 126 while the moisture thereof is removed in the removal unit 127. Accordingly, the humidity of the gas after being heated is reduced to become lower than the humidity of the gas before being heated. Thus, it is possible to prevent the gas from condensing in the suction pipe 124, as well as suppress the measurement unit 121 from being affected by the moisture in the gas. This enables accurate measurement of the particles contained in the gas.

Further, the control unit 123 calculates the expansion of the gas caused by heating, based on the above described Expression 1, to control the suction amount of the pump 122 based on such a calculation result. Accordingly, the measurement unit 121 can obtain the measurement result corresponding to the number of the particles in the predetermined volume of the gas at the predetermined temperature (e.g., 25°C), even when the gas is heated by the heater 126.

When measurement unit 121 is reset to its initial state

As described above, the measurement unit 121 is reset to its initial state once a day. On this occasion, the control unit 123 controls the pump 122 so that the suction amount of the pump 122 becomes greater than the above-described suction amount of the pump 122 when the particles are measured. At this time, the heater 126 heats the gas in the path to the predetermined temperature. Thus, the gas having a high temperature and a humidity lowered by being heated flows into the measurement unit 121, and this dries the interior of the measurement unit 121. Accordingly, reliability of measurement by the measurement unit 121 is ensured.

When humidifying and/or sterilizing interior of chamber 110

As described above, the interior of the chamber 110 is periodically humidified and/or sterilized, to prevent contamination in the interior of the chamber 110 or similar. The interior of the chamber 110 is humidified and/or sterilized such that sterilizing gas is supplied from the supply unit 128 into the chamber 110, or humidifying water and/or sterile liquid is sprayed from the supply unit 128 into the chamber 110. When humidification is performed, measurement of particles is unnecessary, and the control unit 123 stops the suction of the gas by the pump 122 so as to reliably perform humidification and/or sterilization. Note that, after humidification and/or sterilization, in order to remove the humidifying water, sterilizing gas, and sterile liquid from the path of the gas, the control unit 123 may perform control so that the pump 122 sucks the amount of the gas that is greater than the amount of the gas when measurement is performed, similarly to the case of the above described resetting time. Note that, in an embodiment of the present disclosure, the heater 126 is described as having a structure of covering the suction pipe 124, but it is not limited thereto.

In specific, for example, the heater 126 does not only cover the suction pipe 124, but also completely or partially cover the discharge pipe 125.

Further, the gas is returned to the chamber 110 through a flow path on the discharge side, e.g., the discharge pipe 125. Thus, if condensation occurs in such a discharge flow path, water particles may be returned into the chamber 110. Accordingly, it is desirable to retain warmth of the discharge flow path such as the discharge pipe 125.

With such a configuration, it is possible to prevent the gas from condensing I n the discharge pipe 125, as well as suppress the measurement unit 121 from being affected by the moisture in the gas, thereby being able to further accurately measure the particles in the gas.

Further, when a pipe such as the discharge pipe 125 is heated by the heater 126, it is more preferable not to heat the pipe at a temperature higher than about 40°C.

This can secure stability and life of the measurement unit 121, since a sensor used for the measurement unit 121, particularly a laser, is prevented from being adversely affected by high temperature.

[Second Embodiment]

An overall configuration of clean environment equipment 2 according to a second embodiment will be described with reference to FIG. 2. FIG. 2 is a diagram schematically illustrating the clean environment equipment 2 according to the second embodiment. In FIG. 2, the same reference numerals are given to components similar to those in the clean environment equipment 1.

As illustrated in FIG. 2, the clean environment equipment 2 includes, similarly to the clean environment equipment 1 in the first embodiment, a chamber 210, a supply unit 228, and an air particle measurement apparatus 220. However, a component corresponding to the removal unit 127 in the first embodiment is not included.

Further, a heater 226 included in the air particle measurement apparatus 220 partially covers the outside of a housing 229 in a suction pipe 224. Thus, a section covered with the heater 226 in the suction pipe 224 is shorter than the suction pipe 124 in the first embodiment. Accordingly, the heater 226 enables sufficient heating even in the short interval, for example, by heating the gas at a temperature higher than the temperature as in the heater 126 of the first embodiment, and/or increasing the surface area such that the pipe is wound or rolled inside the heater 226.

Such an operation of the clean environment equipment 2 according to the second embodiment is, similarly to the first embodiment, broadly classified into the operation when measuring the particles in the gas, the operation when a measurement unit 221 is reset to its initial state, and the operation when the interior of the chamber 210 is humidified and/or sterilized. The details of these operations are omitted.

Note that, according to the present disclosure, the suction pipe 224 and optionally a discharge pipe 225 are constituted by a hygroscopic resin film. The hygroscopic resin film includes, for example, Nafion tubing which is a widely known member. Although the details are omitted, the Nafion tubing has such a function of removing moisture by discharging the moisture from the inside to the outside of the tube. Note that "NAFION" is a registered trademark.

In specific, such a Nafion tube may be used for the whole or a part of the suction pipe 224. That is, the Nafion tube may be provided in the path between a mounting unit 211 and the heater 226.

With such a configuration, in the path provided with Nafion tube between the mounting unit 211 and the heater 226, moisture is discharged from the Nafion tube, and the gas having a high humidity in the chamber 210 enters the path through the mounting unit 211 and flows toward the heater 226 with its moisture being reduced.

Accordingly, it is possible to prevent the gas from condensing in the suction pipe 224, as well as suppress the measurement unit 221 from being affected by the moisture in the gas, thereby being able to accurately measure the particles in the gas.

Further, the Nafion tube may be used for the whole or a part of the discharge pipe 225. That is, the Nafion tube may be provided in the path between a mounting unit 212 and a pump 222.

With such a configuration, in the path provided with the Nafion tube between the mounting unit 212 and the pump 222, moisture is discharged from the Nafion tube, and the gas with reduced moisture flows from the mounting unit 212 toward the inside of the chamber 210.

Accordingly, it is possible to prevent the gas from condensing in the discharge pipe 225, as well as suppress the measurement unit 221 from being affected by the moisture in the gas, thereby being able to accurately measure the particles in the gas.

[Example]

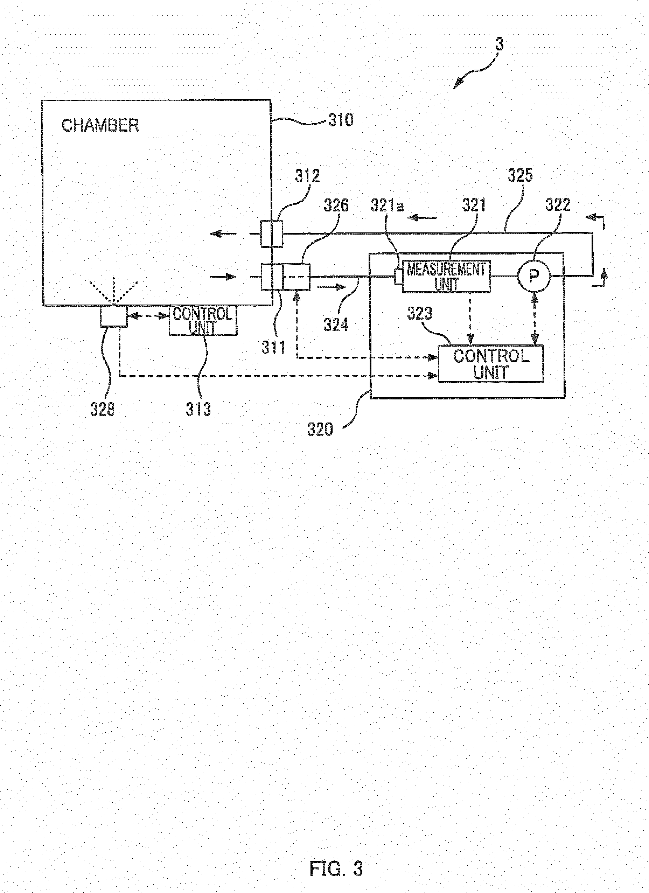

An overall configuration of clean environment equipment according to an example will be described with reference to FIG. 3. FIG. 3 is a diagram schematically illustrating the clean environment equipment according to the example . In FIG. 3, the same reference numerals are given to components similar to those in the clean environment equipment 1.

As illustrated in FIG. 3, the clean environment equipment 3 includes a chamber 310, a supply unit 328, and an air particle measurement apparatus 320. However, similarly to the second embodiment, a component corresponding to the removal unit 127 in the first embodiment is not included.

Further, a heater 326 is mounted to a mounting unit 331 of the chamber 310, and heats the gas flowing through a mounting unit 311 and a suction pipe 324 (connecting portion) . The heater 326 enables sufficient heating, for example, by heating the gas at a temperature higher than the temperature as in the heater 126 of the first embodiment, and/or increasing the surface area such that the pipe is wound or rolled inside the heater 326, similarly to the heater 226 in the second embodiment.

Such an operation of the clean environment equipment 3 according to this example is broadly classified into the operation when measuring the particles in the gas, the operation when a measurement unit 321 is reset to its initial state, and the operation when the interior of a chamber 310 is humidified and/or sterilized, similarly to the first embodiment. The details of these operations are omitted.

The embodiments of the present disclosure are simply to facilitate understanding of the present disclosure and are not in any way to be construed as limiting the present disclosure.

For example, the clean environment equipment is not limited to the uses of regenerative medicine, but may be used for sterile products, powder packing, sterilization test, chemical hazards, and the like.

Further, the discharge pipe 125, 225, may not be provided. In this case, the gas discharged from the pump 122, 222, is discharged to the outside of the clean environment equipment 1, 2. Further, when the gas in the chamber 110, 210, 310 contains a large amount of $CO_2$, for example, it is necessary to supplement $CO_2$ in the chamber 110, 210.

[Reference Signs List]

| 1, 2, 3 | clean environment equipment |
| 110, 210, 310 | chamber |
| 120, 220, 320 | air particle measurement apparatus |
| 121, 221, 321 | measurement unit |
| 122, 222, 322 | pump |
| 123, 223, 323 | control unit |
| 124, 224, 324 | suction pipe |
| 125, 225, 325 | discharge pipe |
| 126, 226, 326 | heater (heating unit) |
| 127 | removal unit |
| 128, 228, 328 | supply unit |

**Claims**

1. An air particle measurement apparatus (120, 220) connected to a chamber (110, 210), the chamber (110, 210) temporarily or constantly maintained at a humidity higher than an outside air humidity, the air particle measurement apparatus (120, 220) comprising:

   a measurement unit (121, 221) configured to measure particles in the drawn gas;
   a suction pipe (124, 224) through which the gas drawn from an interior of the chamber (110, 210) is transported to the measurement unit (121, 221), the suction pipe (124, 224) connecting a mounting unit (111, 211) of the chamber (110, 210) and the measurement unit (121, 221);
   a pump (122, 222) configured to suction the gas so as to be transported from the chamber (110, 210) to the measurement unit (121, 221) through the suction pipe (124, 224); and
   a heating unit (126, 226) configured to heat the gas in a path upstream from the measurement unit (121, 221),
   wherein the suction pipe (124, 224) is formed of a hygroscopic resin or wherein the apparatus comprises a removal unit (127) Z configured to remove moisture in the gas by condensing moisture contained in the gas in the suction pipe (124, 224),
   **characterized in that** the heating unit (126, 226) has a structure to cover the suction pipe (124, 224) upstream from the measurement unit (121, 221).

2. An air particle measurement apparatus (120, 220) according to claim 1, further comprising:

   a discharge pipe (125, 225) through which the gas discharged from the pump (122, 222) is transported to the chamber (110, 210), the discharge pipe (125, 225) connecting the pump (122, 222) and a mounting unit (111, 211) of the chamber (110, 210), wherein the heating unit (126, 226) has a structure to cover the discharge pipe (125, 225).

3. An air particle measurement apparatus (120, 220) Z according to claim 2, wherein the discharge pipe (125, 225) is formed of a hygroscopic resin film.

4. An air particle measurement apparatus (120, 220) according to any one of claims 1 to 3, further comprising:
   a control unit (123, 223) configured to drive the pump (122, 222) so that a flow rate of the gas drawn into the measurement unit (121, 221) when the heating unit (126, 226) is operating to generate a first amount of heat becomes greater than the flow rate when the heating unit (126, 226) is operating to generate a second amount of heat that is smaller than the first amount of heat or the flow rate when the heating unit (126, 226) is stopped.

5. An air particle measurement apparatus (120, 220) according to claim 1, further comprising:

   a control unit (123, 223) configured to control driving of the pump (122, 222) so that a flow rate of the gas drawn into the measurement unit (121, 221) is adjusted, wherein the control unit (123, 223) has
   a first mode of driving the pump (122, 222) so that the flow rate becomes a first flow rate, and a second mode of driving the pump (122, 222) so that the flow rate becomes a second flow rate, the second flow rate being greater than the first flow rate.

6. An air particle measurement apparatus (120, 220) according to claim 1, wherein the removal unit (127) is provided upstream from the heating unit (126, 226).

7. Clean environment equipment (1, 2) configured to be detachably attached with an air particle measurement apparatus (120, 220), the air particle measurement apparatus (120, 220) configured to measure particles in gas, the clean environment equipment (1, 2) comprising:

   a chamber (110, 210) temporarily or constantly maintained at a humidity higher than an outside air humidity;
   a connecting portion to detachably connect the chamber and the air particle measurement apparatus;
   wherein the air particle measurement apparatus (120, 220) is the air particle measurement apparatus (120, 220) according to any one of claims 1 to 6.

8. Clean environment equipment (1,2) according to claim 7, further comprising:

a supply unit (128, 228) configured to supply water vapor or mist-like moisture, to increase a humidity in the chamber (110, 210); and
a control unit (123, 223) configured to drive the pump (122, 222) so that a flow rate of gas drawn into the measurement unit (121, 221) when water vapor or mist-like moisture is supplied from the supply unit (128, 228) becomes smaller than the flow rate when water vapor or mist-like moisture is not supplied from the supply unit (128, 228).

9. Clean environment equipment ; (1, 2) according to claim 8, wherein the control unit (123, 223) stops the pump, (122, 222) when the water vapor or the mist-like moisture is supplied from the supply unit (128, 228).

**Patentansprüche**

1. Luftpartikelmessvorrichtung (120, 220), die mit einer Kammer (110, 210) verbunden ist, wobei die Kammer (110, 210) temporär oder ständig bei einer Feuchtigkeit gehalten wird, die höher ist als eine Außenluftfeuchtigkeit, wobei die Luftpartikelmessvorrichtung (120, 220) aufweist:

eine Messeinheit (121, 221), die ausgebildet ist, Partikel in angesaugtem Gas zu messen;
eine Saugleitung (124, 224), durch die das aus dem Inneren der Kammer (110, 210) angesaugte Gas zu der Messeinheit (121, 221) transportiert wird, wobei die Saugleitung (124, 224) eine Befestigungseinheit (111, 211) der Kammer (110, 210) mit der Messeinheit 121, 221) verbindet;
eine Pumpe (122, 222), die ausgebildet ist, das Gas so anzusaugen, dass es aus der Kammer (110, 210) durch die Saugleitung (124, 224) in die Messeinheit (121, 221) transportiert wird; und
eine Heizeinheit (126, 226), die ausgebildet ist, das Gas in einem Kanal vor der Messeinheit (121, 221) zu erwärmen,
wobei die Saugleitung (124, 224) aus einem hygroskopischen Harz gebildet ist, oder wobei die Vorrichtung eine Entfernungseinheit (127) aufweist, die ausgebildet ist, Feuchtigkeit in dem Gas durch Kondensieren von Feuchtigkeit, die in dem Gas enthalten ist, in der Saugleitung (124, 224) zu entfernen,

**dadurch gekennzeichnet, dass**
die Heizeinheit (126, 226) einen Aufbau hat derart, dass sie die Saugleitung (124, 224) vor der Messeinheit (121, 221) abdeckt.

2. Luftpartikelmessvorrichtung (120, 220) nach Anspruch 1, die ferner aufweist:

eine Auslassleitung (125, 225), durch die das von der Pumpe (122, 222) abgegebene Gas zu der Kammer (110, 210) transportiert wird, wobei die Auslassleitung (125, 225) die Pumpe (122, 222) mit der Befestigungseinheit (111, 211) der Kammer (110, 210) verbindet,
wobei die Heizeinheit (126, 226) einen Aufbau derart hat, dass sie die Auslassleitung (125, 225) abdeckt.

3. Luftpartikelmessvorrichtung (120, 220) nach Anspruch 2, wobei die Auslassleitung (125, 225) aus einer hygroskopischen Harzschicht gebildet ist.

4. Luftpartikelmessvorrichtung (120, 220) nach einem der Ansprüche 1 bis 3, die ferner aufweist:
eine Steuereinheit (123, 223), die ausgebildet ist, die Pumpe (122, 222) derart anzusteuern, dass eine Durchflussrate des in die Messeinheit (121, 221) eingesaugten Gases bei einem Betrieb der Heizeinheit zur Erzeugung einer ersten Wärmemenge größer ist als die Durchflussrate bei Betrieb der Heizeinheit (126, 226) zur Erzeugung einer zweiten Wärmemenge, die kleiner ist als die erste Wärmemenge, oder als die Durchflussrate bei ausgeschalteter Heizeinheit (126, 226).

5. Luftpartikelmessvorrichtung (120, 220) nach Anspruch 1, die ferner aufweist:
eine Steuereinheit (123, 223), die ausgebildet ist, das Ansteuern der Pumpe (122, 222) derart zu steuern, dass eine Durchflussrate des in die Messeinheit (121, 221) angesaugten Gases verstellt wird, wobei die Steuereinheit (123, 223) einen ersten Modus zum Ansteuern der Pumpe (122, 222) derart hat, dass die Durchflussrate zu einer ersten Durchflussrate wird, und einen zweiten Modus zum Ansteuern der Pumpe (122, 222) derart hat, dass die Durchflussrate zu einer zweiten Durchflussrate wird, wobei die zweite Durchflussrate größer ist als die erste Durchflussrate.

6. Luftpartikelmessvorrichtung (120, 220) nach Anspruch 1, wobei die Entfernungseinheit (127) stromaufwärts in Bezug auf die Heizeinheit (126, 226) vorgesehen ist.

7. Vorrichtung für saubere Umwelt (1, 2), die ausgebildet ist, abnehmbar mit einer Luftpartikelmessvorrichtung (120, 220) verbunden zu werden, wobei die Luftpartikelmessvorrichtung (120, 220) ausgebildet ist, Partikel in einem Gas zu messen, wobei die Vorrichtung für saubere Umwelt (1, 2) aufweist:

eine Kammer (110, 210), die temporär oder ständig bei einer Feuchtigkeit gehalten wird, die höher ist als eine Außenluftfeuchtigkeit;
einen Verbindungsbereich zum lösbaren Verbinden der Kammer mit der Luftpartikelmessvorrichtung;
wobei die Luftpartikelmessvorrichtung (120, 220) die Luftpartikelmessvorrichtung (120, 220) nach einem der Ansprüche 1 bis 6 ist.

8. Vorrichtung für saubere Umwelt (1, 2) nach Anspruch 7, die ferner aufweist:

eine Versorgungseinheit (128, 228), die ausgebildet ist, Wasserdampf oder nebelartige Feuchtigkeit zuzuführen, um eine Feuchtigkeit in der Kammer (110, 210) zu erhöhen; und
eine Steuereinheit (123, 223), die ausgebildet ist, die Pumpe (122, 222) derart anzusteuern, dass eine Durchflussrate von Gas, das in die Messeinheit (121, 221) eingesaugt wird, bei Zuführung von Wasserdampf oder nebelartiger Feuchtigkeit durch die Versorgungseinheit (128, 228) kleiner wird als die Durchflussrate ohne Zuführung von Wasserdampf oder nebelartiger Feuchtigkeit aus der Versorgungseinheit (128, 228).

9. Vorrichtung für saubere Umwelt (1, 2) nach Anspruch 8, wobei die Steuereinheit (123, 223) die Pumpe (122, 222) anhält, wenn der Wasserdampf oder die nebelartige Feuchtigkeit aus der Versorgungseinheit (128, 228) zugeführt wird.


**Revendications**

1. Appareil de mesure de particules d'air (120, 220) relié à une chambre (110, 210), la chambre (110, 210) étant temporairement ou constamment maintenue à une humidité supérieure à une humidité d'air extérieur, l'appareil de mesure de particules d'air (120, 220) comprenant :

une unité de mesure (121, 221) configurée pour mesurer des particules dans le gaz prélevé ;
un tuyau d'aspiration (124, 224) à travers lequel le gaz prélevé depuis un intérieur de la chambre (110, 210) est transporté vers l'unité de mesure (121, 221), le tuyau d'aspiration (124, 224) reliant une unité de montage (111, 211) de la chambre (110, 210) et l'unité de mesure (121, 221) ;
une pompe (122, 222) configurée pour aspirer le gaz de manière à être transporté de la chambre (110, 210) jusqu'à l'unité de mesure (121, 221) à travers le tuyau d'aspiration (124, 224) ; et
une unité de chauffage (126, 226) configurée pour chauffer le gaz dans un trajet en amont de l'unité de mesure (121, 221),
dans lequel le tuyau d'aspiration (124, 224) est formé d'une résine hygroscopique ou dans lequel l'appareil comprend une unité d'élimination (127) configurée pour éliminer de la moiteur dans le gaz en condensant de la moiteur contenue dans le gaz dans le tuyau d'aspiration (124, 224),
**caractérisé en ce que**
l'unité de chauffage (126, 226) a une structure pour couvrir le tuyau d'aspiration (124, 224) en amont de l'unité de mesure (121, 221).

2. Appareil de mesure de particules d'air (120, 220) selon la revendication 1, comprenant en outre :

un tuyau de décharge (125, 225) à travers lequel le gaz déchargé depuis la pompe (122, 222) est transporté vers la chambre (110, 210), le tuyau de décharge (125, 225) reliant la pompe (122, 222) et une unité de montage (111, 211) de la chambre (110, 210), dans lequel
l'unité de chauffage (126, 226) a une structure pour couvrir le tuyau de décharge (125, 225).

3. Appareil de mesure de particules d'air (120, 220) selon la revendication 2, dans lequel
le tuyau de décharge (125, 225) est formé d'un film de résine hygroscopique.

4. Appareil de mesure de particules d'air (120, 220) selon l'une quelconque des revendications 1 à 3, comprenant en outre :
une unité de commande (123, 223) configurée pour entraîner la pompe (122, 222) de sorte qu'un débit de gaz prélevé dans l'unité de mesure (121, 221) lorsque l'unité de chauffage (126, 226) est en fonctionnement pour générer une première quantité de chaleur devient supérieur au débit lorsque l'unité de chauffage (126, 226) est en fonctionnement pour générer une seconde quantité de chaleur qui est inférieure à la première quantité de chaleur ou au débit lorsque l'unité de chauffage (126, 226) est arrêtée.

5. Appareil de mesure de particules d'air (120, 220) selon la revendication 1, comprenant en outre :

une unité de commande (123, 223) configurée pour commander l'entraînement de la pompe (122, 222) de sorte qu'un débit du gaz prélevé dans l'unité de mesure (121, 221) est ajusté, dans lequel

l'unité de commande (123, 223) a un premier mode d'entraînement de la pompe (122, 222) pour que le débit devienne un premier débit, et un second mode d'entraînement de la pompe (122, 222) pour que le débit devienne un second débit, le second débit étant supérieur au premier débit.

6. Appareil de mesure de particules d'air (120, 220) selon la revendication 1, dans lequel l'unité d'élimination (127) est agencée en amont de l'unité de chauffage (126, 226).

7. Équipement pour environnement propre (1, 2), configuré pour être fixé de manière amovible à un appareil de mesure de particules d'air (120, 220), l'appareil de mesure de particules d'air (120, 220) étant configuré pour mesurer des particules dans un gaz, l'équipement pour environnement propre (1, 2) comprenant :

   une chambre (110, 210) maintenue temporairement ou constamment à une humidité supérieure à une humidité d'air extérieur ;
   une partie de connexion pour connecter de manière amovible la chambre et l'appareil de mesure de particules d'air ;
   dans lequel l'appareil de mesure de particules d'air (120, 220) est l'appareil de mesure de particules d'air (120, 220) selon l'une quelconque des revendications 1 à 6.

8. Équipement pour environnement propre (1, 2) selon la revendication 7, comprenant en outre :

   une unité d'alimentation (128, 228) configurée pour alimenter de la vapeur d'eau ou de la moiteur de type brouillard, pour augmenter une humidité dans la chambre (110, 210) ; et
   une unité de commande (123, 223) configurée pour entraîner la pompe (122, 222) de sorte qu'un débit de gaz prélevé dans l'unité de mesure (121, 221) lorsque de la vapeur d'eau ou de la moiteur de type brouillard est alimentée par l'unité d'alimentation (128, 228) devient inférieur au débit lorsque de la vapeur d'eau ou de la moiteur de type brouillard n'est pas alimentée par l'unité d'alimentation (128, 228).

9. Équipement pour environnement propre (1, 2) selon la revendication 8, dans lequel l'unité de commande (123, 223) arrête la pompe (122, 222) lorsque la vapeur d'eau ou la moiteur de type brouillard est alimentée par l'unité d'alimentation (128, 228).

FIG. 1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006058239 A
- EP 2692851 A2
- EP 0976268 A1

- JP 2010154792 A
- JP 2009002711 A
- US 2010313963 A1